# EUROPEAN PATENT SPECIFICATION

(11) **EP 3 670 362 B1**
(45) Date of publication and mention of the grant of the patent: **15.06.2022**
(21) Application number: 18215421.1
(22) Date of filing: 21.12.2018
(51) Int. Cl.: B64G 1/48, B01D 11/02, B01D 53/62, B01D 53/02, A61L 2/16, B01D 11/04

(54) **CLOSED ENVIRONMENTAL COMPARTMENT TO ACCOMMODATE HUMANS**
GESCHLOSSENER UMGEBUNGSRAUM ZUR AUFNAHME VON MENSCHEN
COMPARTIMENT ENVIRONNEMENTAL FERMÉ POUR ACCOMMODER LES HUMAINS

(43) Date of publication of application: 24.06.2020
(73) Proprietor: Airbus Defence and Space GmbH, 82024 Taufkirchen (DE)
(72) Inventor: Janecek, Vladislav, 88213 Ravensburg (DE)
(74) Representative: Daub, Thomas

(56) References cited:
- WO-A1-89/08659
- WO-A1-90/06189
- WO-A1-03/063998
- WO-A2-2007/027876
- RU-C1- 2 500 590
- US-A1- 2003 150 232
- Nasa: "NASA Technology Roadmaps TA 6: Human Health, Life Support, and Habitation Systems", , 1 July 2015 (2015-07-01), XP055580664, Retrieved from the Internet: URL:https://www.nasa.gov/sites/default/fil es/atoms/files/2015_nasa_technology_roadma ps_ta_6_human_health_life_support_habitati on_final.pdf [retrieved on 2019-04-12]

## Description

The invention relates to a spacecraft.

Closed inhabited compartment ecosystems such as a spacecraft requires extremely efficient resources management. The external supply has to be minimized as it represents logistical challenges and consequently costs. Such topic is a very relevant with respect to International Space Station, planned Lunar Orbital Platform or future extraterrestrial colonization in deep space missions. For the latter, a crew has to be equipped with multifunctional systems allowing to deal autonomously with expected but also unexpected problems and situations.

A closed compartment inhabited by a crew necessarily requires environmental control and life support system. Such a system covers life support needs and deals with human metabolic products such as carbon dioxide CO₂. One crew member produces about 1 kg of CO₂ per day. The carbon dioxide concentration has to be kept under critical value and thus has to be continuously removed from surroundings. Practically all commonly used technics at some point deal with concentrated carbon dioxide CO₂ which is either further processed, or stored or vented. However, carbon dioxide CO₂ can be advantageously used for many important processes contributing to long term sustainability.

The object of the invention is in particular to provide a spacecraft with improved utilization of carbon dioxide. The object is achieved by the features of claim 1, while advantageous embodiments and variations of the invention can be taken from the dependent claims.

RU 2 500 590 C1 shows a regenerative physical-chemical maximum closed life support system of crews of durable spacecraft. The regenerative system comprises a habitable module, a system to remove harmful impurities, a system to remove carbon dioxide including adsorbent and heaters, a carbon dioxide concentrator including adsorbent, and a carbon dioxide and hydrogen processing system including heaters.

WO 90/06189 A1 shows a process for removing two or more contaminants from a substrate in a single process.

WO 03/063998 A1 shows a method for cleaning of hydrocarbon-containing materials containing solids and/or liquids which involves contacting the materials with an extracting fluid under conditions of temperature and pressure sufficient to maintain the fluid at, near or above its critical point and to products derived therefrom.

The disclosure "NASA Technology Roadmaps TA 6": Human Health, Life Support, and Habitation Systems; www.nasa.gov; page 118-119 shows an alternative CO2 sorbent system.

WO2007/027876 A3 shows a system for oxygen, hydrogen and carbon mass regeneration and recycling for breathing, and fuel/energy generation purposes, especially for fuel cells and rocket motors, by combination and integration of a photoelectrolytically powered electrochemical and gas handling system with one or more fuel cells.

### Advantages of the invention

The invention proposes a closed environmental compartment with a life support system configured to remove metabolic carbon dioxide from the compartment's interior atmosphere, and with at least one work chamber, which is configured for a performance of an extraction and/or purification process with carbon dioxide as a solvent, wherein the carbon dioxide required for the extraction and/or purification process in the work chamber is supplied by the life support system. The work chamber is preferably connected via lines directly to a line system, in particular for the transport of carbon dioxide, of the life support system. Preferably, the work chamber is coupled via lines and/or conditioning means directly to the life support system. In particular, a direct, automatic transport of a carbon dioxide from the life support system to the work chamber takes place in one operation. The work chamber is preferably coupled to the life support system independently of an operating state of the work chamber.

A "closed environmental compartment", is to be understood in this context in particular as a man-made habitat for at least one human being. Such closed environmental compartments do regularly not rely on any matter exchange with any part outside the compartment. There are various conceivable closed environmental compartments suitable for a person skilled in the art, such as spacecraft, mobile space stations like the ISS International Space Station, immobile extraterrestrial space stations, e.g. dwellings on a planet or a moon, but also submarines.

In this context, a "life support system" is to be understood as, in particular, a sub-system of a closed environmental system which results in the provision and recovery of at least one vital substance, in particular oxygen, water and/or carbon in a cabin and/or the removal of harmful substances, in particular carbon dioxide, preferably metabolic carbon dioxide, from the interior of the closed environmental system. Preferably, the provision and recovery and/or a removal take place by means of an at least partially closed circuit. For example, the life support system may be based on physicochemical processes. Particularly preferably, the air renewal system is provided for at least partial provision and recovery of oxygen and removal of carbon dioxide.

Furthermore, in this context, a "work chamber" should be understood to mean in particular a reaction and/or purification chamber. The work chamber preferably provides a separate space, in particular closed in at least one operating state, for performing an extraction and/or purification process. Preferably, the work chamber is provided in particular for a direct accommodation to the materials or objects (e.g. garments) that are to be subjected to the extraction and/or purification process. Various extraction and/or purification processes that appear appropriate to a person skilled in the art are conceivable. Generally, an extraction or purification process within the meaning of the present invention is any process where one component (the extractant) is separated from another component using carbon dioxide as a solvent in a supercritical or near-supercritical state. The work chamber can be provided, for example, for carrying out purification processes of fabrics, garments, dishes, delicate mechanical or structural components. In particular, a universal washing device can be implemented. Furthermore, the work chamber may for example be provided for the implementation of disinfection processes for various materials and objects, such as surgical material, laboratory equipment, experiment samples, sensitive devices such as endoscopes or the like. Furthermore, the work chamber may for example be provided for terminal sterilization at low temperature with very short treatment time. Moreover, the work chamber may be provided for extraction, for example for food processing, decontamination, waste treatment or the like. In particular, the carbon dioxide CO₂ at a supercritical state can be used as a solvent for the extraction. The application can be for example a mineral oil, which is spoiled on Kevlar textile. By means of the supercritical carbon dioxide CO₂ treatment one can remove the mineral oil from the textile. In addition, the oil can be even recovered and further reused. Furthermore, the extraction and/or purification system can be provided, for example, for the performance of extraction processes, for example waste treatment processes using supercritical or near-supercritical carbon dioxide. Furthermore, a combination of different processes for forming the extraction and/or purification system as a multi-product platform would also be possible. "Provided" is in particular to mean specifically programmed, designed and/or equipped. By an object being provided for a certain function is in particular to be understood that the object fulfills and/or implements said certain function in at least one application state and/or operating state.

In particular, the work chamber forms part of a closed process loop for processing and handling the carbon dioxide CO₂. Such a system typically consists of an internal vessel, a pump, temperature conditioning elements, the work chamber, pressure reducing device, selective separator unit, in particular for separation of carbon dioxide CO₂ and waste or substances to be extracted, for example water. As the system operates preferably in a closed loop, a majority of CO₂ can be reused within the device. In practice, however, losses of carbon dioxide are generally unavoidable (e.g. small losses may occur while opening the work chamber in between consecutive washing cycles). The present invention provides an advantageous solution of how to supply carbon dioxide in order to compensate for the losses occurred during normal operation of the system by connecting the work chamber to the life support system as a source of carbon dioxide.

By the inventive implementation a meaningful reuse of carbon dioxide from the life support system can be achieved. In particular, the carbon dioxide of the life support system, which in particular has to be discharged at least partially, can be used for an extraction and/or purification process. In this way a sensible use of the carbon dioxide can be achieved. Furthermore, at the same time, in particular continuous, provision of carbon dioxide for the work chamber can be achieved. In particular, the use of separate gas tanks can be dispensed with.

Carbon dioxide CO₂ itself is a stable, colorless, noncorrosive, non-flammable and inert gas. It is a versatile industrial substance on Earth used for many purposes for example as an inert gas for welding, fire extinguishers, solvent, dry ice blasting or energy vector. Its potential is however not fully exploited since CO₂ based process equipment requires relatively large development effort. On the other hand, under limited resources considerations such as in a spacecraft or space station, CO₂ based processes are an elegant solution allowing high efficiency and variability. It is thus possible to develop a versatile and multi-functional platform.

It is also according to the invention that the closed environmental compartment comprises a further work chamber, which is configured for a performance of an extraction and/or purification process with carbon dioxide as a solvent, and which is directly connected to the life support system, at a substantial distance from the work chamber. In particular, a multiplicity of work chambers, in particular with different functions, can be connected to the life support system. The life support system can provide carbon dioxide to the work chambers at a variety of locations inside the spacecraft. In particular, an existing line system of the life support system can be used to supply the work chambers with carbon dioxide. The work chambers may in particular be of identical design, or can be provided for carrying out various extraction and/or purification processes, wherein in particular in each process carbon dioxide is used as a solvent. As a result, an advantageously variable use of the output of the life support system can be achieved.

It is further according to the invention that the life support system comprises a CO₂ absorbent material for CO₂ absorption, in particular for CO₂ binding. Preferentially, the life support system is configured to convey the ambient air of the closed environmental compartment's interior at least partly through the CO₂ absorbent material for the purpose of binding the carbon dioxide present in the ambient air in the CO₂ absorbent material. In the context of the present invention "ambient air" is to be understood as the mixture of gases that form the atmosphere inside the closed environmental compartment. This mixture is usually different from the mixture of gases that form the atmosphere on the surface of the Earth and may exhibit a different pressure level as compared to atmospheric pressure. The life support system is configured to regenerate the CO₂ absorbent material following a CO₂ binding, for the purpose of removing the carbon dioxide from the CO₂ absorbent material. According to the invention, the life support system is provided to supply the separated, carbon dioxide to a pipeline system that forms part of the carbon dioxide distribution network within the closed environmental compartment.

There are several direct carbon dioxide separation technologies suitable for use according to the present invention. An absorber with a solid sorbent, like Astrine^{™} of Airbus Defence and Space, Taufkirchen, Germany, is used successfully in advanced closed loop life support systems for space vehicles and submarines. Another example is the known usage of aqueous hydroxide sorbent used to produce carbon-free gasoline.

It is proposed according to the invention that the CO₂ absorbent material is implemented as a macro-porous ion-exchange resin, in particular according to the one disclosed according to DE19830470C1. Preferably the CO₂ absorbent material is implemented as a macro-porous ion-exchange resin with vinyl benzene polymers, which are polymerized with divinylbenzene and comprise primary benzylamines as functional groups. Resins comprising primary benzylamine groups have a particularly high binding capability for gaseous CO₂. The intake capacity of the resin for CO₂ is under normal atmospheric conditions, in particular in case of saturation, at least 60 g CO₂ per kg resin referring to the dry mass of the resin. Preferred parameters of an ion-exchange resin that is to be used are a polymerization grade of 2 % to 10 %, a concentration of the functional groups between 2 and 3 mol/l, porosity between 20 % and 30 %, and a middle pore diameter between 200 and 300 angstrom. In an advantageous implementation of the life support system the ion-exchange resin is exposed to the CO₂-containing ambient air by way of the air being conveyed through a ion-exchange resin in bulk form by means of an air pump, preferably a blower. When flowing through the resin bulk the CO₂ molecules are bound to the functional primary benzyl amine groups on the outer and inner surfaces of the macro-porous resin beads and the medium flowing through is correspondingly depleted. The regeneration of the resin may be effected in a plurality of ways; a selection of the type or regeneration is herein dependent on the application case at hand and other technical and logistic boundary conditions. A regeneration of the ion-exchange resin is effected by a usage of slightly overheated water vapor under atmospheric conditions and the resulting expulsion of the CO₂. Alternatively the regeneration of the ion-exchange resin is effected by applying a negative pressure. In this way in particular a separation of carbon dioxide from an ambient air is reliably achievable. This in particular allows a reliable supply of carbon dioxide. In particular, a selected extraction of carbon dioxide from an ambient air is achievable.

Moreover it is proposed that the spacecraft comprises a transfer unit, which is configured to transferring the carbon dioxide of the life support system into a supercritical or at least near-supercritical state before being introduced into the work chamber. Preferably, the transfer unit is configured for transferring the carbon dioxide of the life support system into a supercritical or at least near-supercritical state for a performance of an extraction and/or purification process in the work chamber. A "supercritical fluid" is in particular any substance at a temperature and pressure above its critical point. In such a supercritical state, the fluid exhibits properties between gas and liquid properties. In particular, supercritical liquids possess a liquid-like density, a gas-like viscosity and surface tension, and diffusivities in a range intermediate between those of a liquid and a gas. Such substances diffuse through solids like a gas, and dissolve materials like a liquid. Generally, supercritical fluids are suitable as substitutes for organic solvents, they can be used for sterilization, extraction of substances and most importantly for purification. Carbon dioxide in its supercritical or at least near-supercritical state is in particular in a fluid state above its critical temperature and above its critical pressure or near its critical temperature and near its critical pressure. "Near" is in particular to mean, in this context, that a value of a temperature or of a pressure is at least 70 %, preferably at least 80 %, preferentially at least 90 % and particularly preferably at least 90 % of a critical value. In a supercritical state the characteristics of carbon dioxide are in particular between the characteristics of a gas and a liquid, supercritical carbon dioxide having in particular the same density as a liquid but the same viscosity as a gas. Supercritical carbon dioxide is generated in particular at a temperature of more than 31.1°C and at a pressure of more than 7.375 MPa (73.75 bar). The critical molar volume of supercritical carbon dioxide is in particular 94 cm³ mol⁻¹. The transfer unit is in particular configured to transfer carbon dioxide of the life support system into a defined state. The transfer unit is in particular configured to transfer carbon dioxide of the life support system into a supercritical state. The transfer unit preferentially comprises dedicated means for the manipulation of pressure and temperature of the carbon dioxide, preferably at least at least one pump and at least one heating unit, which are in at least one operation state configured to bring the carbon dioxide into a supercritical or at least near-critical state. It is in this way in particular possible to advantageously provide supercritical or at least near-critical carbon dioxide for an extraction and/or purification process. It is in particular possible to directly generate supercritical or at least near-critical carbon dioxide for an extraction and/or purification process. Furthermore, carbon dioxide has in its supercritical state approximately zero viscosity and surface tension. It is thus capable of easily entering into cavities or textiles without any additional chemicals. In classical water-cleaning technology a similar effect can only be achieved by adding chemical substances to water. As a conclusion supercritical carbon dioxide CO₂ with universal solvent properties can be used to design a multi-product platform of separators and cleaners as one or several, modular systems. Such a system is useful in particular for space application especially for long term missions.

It is also proposed that the at least one work chamber is configured for a usage of the carbon dioxide in a supercritical or at least near-critical state. Preferentially the work chamber is configured, for an extraction and/or purification process, to feed in carbon dioxide in a supercritical or at least near-supercritical state as a solvent. This in particular allows advantageously using supercritical or at least near-supercritical carbon dioxide for an extraction and/or purification process of the work chamber.

In addition, it is proposed that the spacecraft comprises at least one storage unit, which is arranged upstream of the work chamber and is configured for an intermediate storage of the carbon dioxide. Preferably the storage unit in particular implements a temporary storage, which is configured, before and/or during an extraction and/or purification process, for an intermediate storage of generated carbon dioxide. The storage unit in particular serves as a buffer storage for the extraction and/or purification process. It would moreover also be conceivable that the storage unit is configured for the storage of carbon dioxide of a previous extraction and/or purification process. Preferentially, the carbon dioxide for the extraction and/or purification process is separated via the carbon dioxide separation device before and/or during the extraction and/or purification process, intermediately stored via the storage unit and then - according to demand - processed by the transfer unit. Preferably the storage unit is in particular implemented as a storage tank, in particular a liquid and/or gas tank. Particularly preferably the storage unit is implemented as a liquid tank, which is configured to accommodate the carbon dioxide in a liquid phase or state of matter. The transfer unit is fluidically arranged in particular downstream of the storage unit. In this way in particular an intermediate storage of carbon dioxide is achievable. It is in particular possible to achieve a buffering of carbon dioxide for an extraction and/or purification process. By means of the storage unit it is in particular possible to compensate for a discontinuous supply of carbon dioxide by the life support system. Furthermore, by means of the storage unit carbon dioxide, in particular recovered carbon dioxide, of a last extraction and/or purification process can be stored for the next extraction and/or purification process.

The carbon dioxide stream that exits the working chamber will be contaminated by the extractant (i.e. the component that was extracted during the purification or extraction process). Accordingly, it is proposed that the spacecraft comprises at least one carbon dioxide recovery unit, which is configured for an at least partial separation of carbon dioxide from the contaminated carbon dioxide stream exiting the work chamber. Preferably the carbon dioxide recovery unit is configured for a separation of pure carbon dioxide out of a contaminated carbon dioxide of the work chamber. It is in particular possible that the separated carbon dioxide is discharged and/or particularly preferably that it is re-conveyed to a storage unit, in particular the storage unit described above, for re-use. As regards the separated contaminations (i.e. the extractants), they may be collected and advantageously used for suitable other purposes. By a "carbon dioxide recovery unit" is in particular, in this context, a separation unit to be understood which is configured for a selective extraction of pure carbon dioxide from a contaminated carbon dioxide flow. Preferably the device is configured for a selective separation of carbon dioxide from a fluid stream exiting the work chamber. Different methods, deemed expedient by someone skilled in the art, for a separation of carbon dioxide from a contaminated carbon dioxide stream can be used. A separation may, for example, be effected by an absorber and/or by separating off contaminations. In another implementation, a separation corresponding to the life support system may be effected by an absorber, in particular by an ion-exchange resin. The absorber is herein in particular configured for binding the carbon dioxide, wherein a carbon dioxide is preferably removable from the absorber via a regeneration process. This in particular allows achieving a selective separation of carbon dioxide from a contaminated carbon dioxide stream exiting the work chamber. In this way in particular a re-use of the carbon dioxide is achievable. This further allows in particular achieving a selective disposal of contaminations. In a preferred implementation, the separation of the extractants from the carbon dioxide is done by reducing pressure. The carbon dioxide will immediately turn into gas state and thereby the gaseous carbon dioxide and the solid or liquid extractants are separated.

The invention is furthermore based on a method according to claim 6.

It is moreover proposed that the carbon dioxide is in a supercritical or at least near-supercritical state during the extraction and/or purification process. Preferably the carbon dioxide is brought into a supercritical or at least near-supercritical state for the extraction and/or purification process by means of the transfer unit. Preferably the supercritical or at least near-supercritical carbon dioxide acts as a solvent during the extraction and/or purification process. This in particular allows advantageous usage of supercritical or at least near-supercritical carbon dioxide for an extraction and/or purification process. In this way an advantageous extraction and/or purification result is achievable.

According to the invention, it is further proposed that the provision of carbon dioxide by the life support system is performed by exposing the ambient air of the closed environmental compartment's interior to carbon dioxide absorbent material. Preferably, the ambient air is conveyed to a CO₂ absorbent material for a CO₂ binding. The CO₂ absorbent material is then in particular regenerated for the purpose of removing the carbon dioxide from of the CO₂ absorbent material. The CO₂ absorbent material is preferably implemented of a macro-porous ion-exchange resin. Preferentially the CO₂ absorbent material is implemented of a macro-porous ion-exchange resin with vinylbenzene polymers, which are polymerized with divinylbenzene and contain primary benzylamines as functional groups. In this way in particular a separation of carbon dioxide from an ambient air inside the closed environmental compartment is reliably achievable. It is in this way in particular possible to achieve a reliable supply of carbon dioxide. In particular a selective extraction of carbon dioxide from the ambient air is achievable.

The extraction and/or purification system according to the invention and the method are herein not to be restricted to the application and implementation described above. In particular, for the purpose of fulfilling a functionality that is described here, the extraction and/or purification system according to the invention and the method may comprise a number of respective elements, structural components and method steps that differs from a number given here. Moreover, as regards value ranges given in the present disclosure, values within the limits named are to be considered as disclosed and as applicable as required.

### Drawings

Further advantages will become apparent from the following description of the drawings. The drawings show an exemplary embodiment of the invention. The drawings, the description and the claims contain a plurality of features in combination. Someone skilled in the art will purposefully also consider the features individually and will find further expedient combinations.

It is shown in:
- Fig. 1: a spacecraft, as one particular embodiment of a closed environmental compartment according to the invention comprising a passenger cabin, a life support system and a work chamber in a schematic presentation,
- Fig. 2: the life support system and the work chamber of the spacecraft in a schematic presentation and
- Fig. 3: a schematic flow chart of a method for an operation of the spacecraft according to the invention.

### Description of the exemplary embodiment

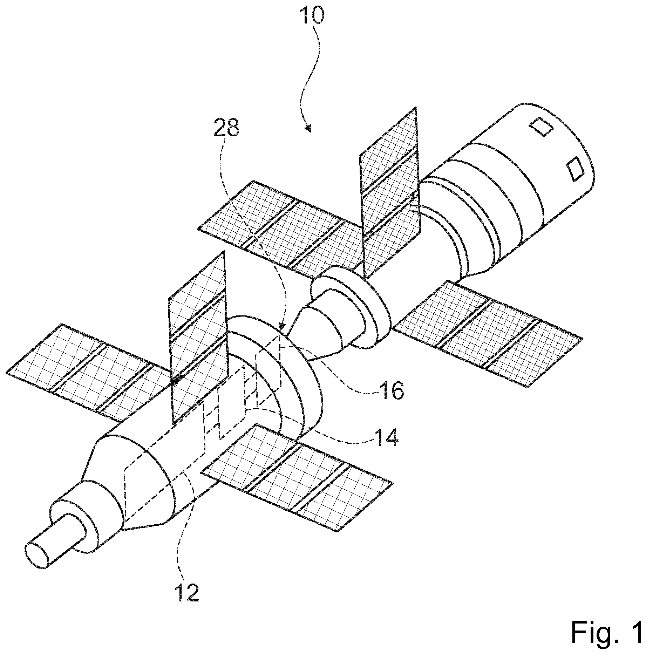

Figure 1 shows a closed environmental compartment, embodied as a manned spacecraft 10, in this case a space station. The spacecraft 10 is implemented as a space station. In principle, however, would also be another implementation of the closed environmental compartment possible, which is conceivable for a person a skilled in the art. The spacecraft 10 is intended for use in space under conditions of reduced or increased gravity. Furthermore, the spacecraft 10 comprises a passenger cabin 12. The passenger cabin 12 is provided for accommodating a crew. The passenger cabin 12 consists of at least one room, but may also consist of several interconnected rooms. The rooms can be connected to each other both directly and via locks. The passenger cabin 12 is at least substantially closed relative to an environment in at least one operating state.

Furthermore, the spacecraft 10 has a life support system 14. The life support system 14 is provided for the spacecraft 10. The life support system 14 is intended to extract from the passenger cabin 12 ambient air with metabolic carbon dioxide CO₂ therein. The life support system 14 is at least partially intended to recover oxygen from the ambient air of the passenger cabin 12 and supply the recovered oxygen O₂ to the passenger cabin 12. The ambient air is sucked off, for example, by means of a not further visible pump or fan.

Moreover, the life support system 14 is configured to remove metabolic carbon dioxide CO₂ from the passenger cabin 12. The life support system 14 is configured for a supply of separated carbon dioxide CO₂ from the ambient air of the passenger cabin 12. The life support system 14 is configured for a separation of carbon dioxide CO₂ from a fluid stream of the ambient air of the passenger cabin 12. The life support system 14 is configured for an extraction of a portion of an ambient air of the passenger cabin 12 in a separate fluid stream, and to extract carbon dioxide CO₂ from the fluid stream by means of an absorber. The life support system 14 comprises a CO₂ absorbent material for carbon dioxide CO₂ absorption. The CO₂ absorbent material is suitable for CO₂ binding. The CO₂ absorbent material is implemented as a macro-porous ion-exchange resin. The CO₂ absorbent material is implemented as a macro-porous ion-exchange resin with vinylbenzene polymers, which are polymerized with divinylbenzene and contain primary benzylamine as functional groups.

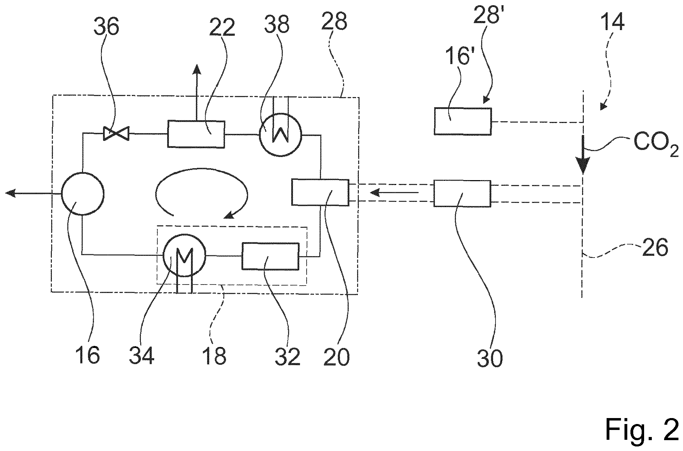

The carbon dioxide separation device 12 is configured to convey the ambient air of the passenger cabin 12 through the CO₂ absorbent material for the purpose of binding carbon dioxide CO₂ at the CO₂ absorbent material. The ion-exchange resin is herein in particular exposed to the CO₂-containing ambient air of the passenger cabin 12 by way of the air being conveyed through a ion-exchange resin bulk by means of an air pump, preferably a blower. When flowing through the resin bulk material, the CO₂ molecules are bound to the functional primary benzylamine groups on the outer and the inner surfaces of the macro-porous resin beads, and the medium flowing through is depleted accordingly. The life support system 14 is configured to regenerate the CO₂ absorbent material after a CO₂ binding, for the purpose of removing-solving the carbon dioxide CO₂ from of the CO₂ absorbent material and then conveying it onwards. A regeneration of the ion-exchange resin is effected, for example, by an application of slightly overheated water vapor under atmospheric conditions and by the expulsion of the carbon dioxide CO₂resulting therefrom. After removing the carbon dioxide CO₂ from of the CO2 absorbent material, the carbon dioxide CO₂ is supplied to a pipeline system 26 of the life support system 14, which is intended to distribute the carbon dioxide CO₂ in the spacecraft 10. In particular, the pipeline system 26 forms a CO₂ network of the spacecraft (fig. 2). The CO₂ network is part of an infrastructure, connecting CO₂ users with the life support system 14 producing carbon dioxide CO₂.

Furthermore, the spacecraft 10 comprises a CO₂ processing subsystem 28 shown in figure 2. The spacecraft 10 comprises at least one work chamber 16, which is configured for a performance of a purification process with carbon dioxide CO₂ as a solvent. The work chamber 16 forms part of the CO₂ processing system 28. The CO₂ processing system 28 is configured for a performance of a purification process. In principle, however, it would also be conceivable that the CO₂ consuming system 28 is configured for an extraction process. The CO₂ processing system 28 may be configured, for example, for a performance of purification processes, e.g. of fabrics, garments, dishes, delicate mechanical or structural component, or the like using supercritical or near-supercritical carbon dioxide. The CO₂ processing system 28 may further be configured, for example, for a performance of disinfection processes, e.g. of surgical instruments, laboratory equipment, or the like. Moreover, the CO₂ processing system 28 may be configured, for example, for the performance of extraction processes, for example waste treatment processes. The CO₂ processing system 28 comprises a control and regulation unit (not shown), which is configured to control a purification process.

The CO₂ processing system 28 can be categorized as a sort of washing machine using supercritical carbon dioxide instead of water. Such sort of washing machine itself is a very sustainable and gentle way of cleaning. It does not require any chemical process enhancers. Garments are rendered free of odor, do not need to undergo drying and the consumed energy is lower than that of conventional cleaning. Such technique is already known from US 4,012,194 or US 5,467,492 A. The CO₂ processing system 28 can be in addition used for selective extraction of particular substances from tissue or objects and subsequently reuse them for particular purposes for example as fertilizers.

The carbon dioxide CO₂ required for the purification process in the work chamber 16 is supplied by the life support system 14. The CO₂ processing system 28 is therefore coupled with the pipeline system 26 of the life support system 14. The CO₂ processing system 28 can receive carbon dioxide CO₂ from the pipeline system 26 via a valve (not shown), controlled by the control and regulation unit, as needed. The spacecraft 10 comprises a CO₂ conditioning unit 30 which is arranged between the CO₂ processing system 28 and the pipeline system 26. The conditioning unit 30 is connected to the pipeline system 26 of the life support system 14 via a line. Carbon dioxide CO₂ from the pipeline system 26 separated off by means of the life support system 14 is conveyed to the conditioning unit 30. The conditioning unit 30 is configured for a conditioning of the carbon dioxide CO₂ in terms of humidity, pressure and other parameters according to operational requirements of the purification process in work chamber 16. Carbon dioxide CO₂ removed from the pipeline system 26 is supplied to the conditioning unit 30 prior to delivery to the CO₂ processing system 28. Following a conditioning the carbon dioxide CO₂ is fed to a storage unit 20 within the CO₂ processing system 28. The spacecraft 10 comprises the storage unit 20. The storage unit 20 is connected with the conditioning unit 30 via a line. The storage unit 20 is arranged upstream of the work chamber 16. The storage unit 20 is configured for an intermediate storage of the carbon dioxide CO₂. The storage unit 20 implements a temporary storage for an intermediate storage of carbon dioxide CO₂. The storage unit 20 serves as a buffer storage for a purification process of the CO₂ processing system 28. Thereby, the purification process performed in work chamber 16 is decoupled from the supply of carbon dioxide via the life support system 14. The storage unit 20 is further configured for the storage of carbon dioxide CO₂ recovered from a previous purification process of the CO₂ processing system 28. The storage unit 20 is embodied by a storage tank. The storage unit 20 is embodied by a liquid tank that is configured to accommodate the carbon dioxide CO₂ in a liquid aggregation state.

The spacecraft 10 furthermore comprises a transfer unit 18. The transfer unit 18 is provided to transfer the carbon dioxide CO₂ of the life support system 14 into a supercritical state. The transfer unit 18 is configured to transferring the carbon dioxide CO₂ of the life support system 14 into a supercritical state before being introduced into the work chamber 16. For this purpose, the transfer unit 18 is connected to the storage unit 20 via a line. Via a not shown valve, which is controlled by the control unit of the spacecraft 10, the transfer unit 18 is supplied as needed with carbon dioxide CO₂. The transfer unit 18 is provided to transfer the carbon dioxide CO₂ in a supercritical state for the work chamber 16 to perform a purification process. Carbon dioxide CO₂ in a supercritical state is in a fluid state above its critical temperature and above its critical pressure. The transfer unit 18 comprises a pump 32. The pump 32 is intended to increase a pressure of carbon dioxide CO₂ above a critical value. The pump 32 is designed to compress the carbon dioxide CO₂ to a pressure of more than 7.375 MPa (73.75 bar). Furthermore, the transfer unit 18 comprises a heating unit 34. The heating unit 34 is provided to raise a temperature of the carbon dioxide CO₂, in particular the already compressed carbon dioxide CO₂, above a critical value. The heating unit 34 is designed to heat the carbon dioxide CO₂ to a temperature higher than 31.1 °C. The heating unit 34 is arranged fluidically behind the pump 32. In principle, however, an alternative arrangement of the heating unit 34 and the pump 32 would be conceivable.

The spacecraft 10 further comprises the work chamber 16. The work chamber 16 is provided for carrying out a purification process with carbon dioxide CO₂ as a solvent. In principle, however, it would also be conceivable that the work chamber 16 is provided for carrying out an extraction process. An input side of the work chamber 16 is connected to the transfer unit 18 via a line. In operation, the work chamber 16 can be supplied via the transfer unit 18, the storage unit 20 and the conditioning unit 26 with carbon dioxide CO₂ of the pipeline system 26 of the life support system 14. The work chamber 16 is configured for a usage of carbon dioxide CO₂ in a supercritical or near-supercritical state for the performance of the purification process. The work chamber 16 can be manually and/or automatically charged via a non-visible access opening with materials or objects (e.g. garments) that are to be purified before the purification process is started and before the carbon dioxide CO₂ is introduced. During the purification process, the work chamber 16 is closed.

After a purification process, contaminated carbon dioxide CO₂ is discharged at an exit side of the work chamber 16. The output side of the work chamber 16 is connected via a line to a reducing valve 36. The reducing valve 36 is provided for lowering a pressure of the contaminated carbon dioxide, in particular below a critical value.

Furthermore, the spacecraft 10 has a carbon dioxide recovery unit 22. The carbon dioxide recovery unit 22 is connected via a line to the reducing valve 36. The carbon dioxide recovery unit 22 is arranged downstream of the work chamber 16. The carbon dioxide recovery unit 22 is provided for separating carbon dioxide CO₂ from the contaminated carbon dioxide stream leaving the work chamber 16. The carbon dioxide recovery unit 22 is intended to separate pure carbon dioxide CO₂ from the contaminated carbon dioxide of the work chamber 16. The contaminations thus removed may be collected and advantageously prepared for suitable other purposes, e.g. as a fertilizer. The separated, pure carbon dioxide CO₂ is supplied to the storage unit 20 for reuse. The separated, pure carbon dioxide CO₂ is, via a cooler 38, supplied via a line to the storage unit 20. The cooler 38 serves in particular for the conditioning of the carbon dioxide CO₂ before entering the storage unit 20.

The storage unit 20, the transfer unit 18, the work chamber 16, the carbon dioxide recovery unit 22 and the cooler 38 each form part of the CO₂ processing system 28. Furthermore, it would be possible that the spacecraft 10 comprises a further work chamber 16', in particular a further CO₂ processing system 28', which is configured for a performance of an extraction and/or purification process with carbon dioxide CO₂ as a solvent, and which is directly connected to the life support system 14, at a substantial distance from the work chamber 16. A multiplicity of work chambers 16, 16', in particular with different functions, can be connected to the life support system 14. The work chambers 16, 16' may in particular be of identical design, or can be provided for carrying out various extraction and/or purification processes, wherein in particular in each process carbon dioxide CO₂ is used as a solvent.

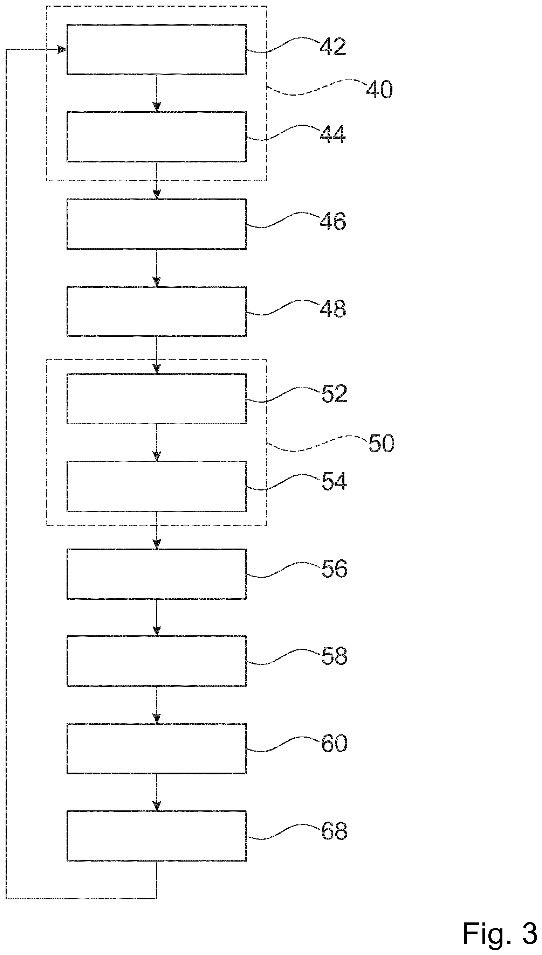

Figure 3 shows a schematic flow diagram of a method for operating the spacecraft 10. The method describes a purification process of a material or object to be cleaned. The process is capable of being performed according to demand. Preferably, the cleaning process is started by a human operator, but it would also be conceivable that the cleaning process is carried out completely automated. Preferably, at the beginning of the process, the work chamber 16 is filled with the cleaning material.

The method comprises the step of providing carbon dioxide CO₂ by means of the life support system 14. The provision of carbon dioxide CO₂ by the life support system 14 is performed by exposing ambient air of the internal atmosphere of the closed environmental compartment, e.g. of the passenger cabin 12, to carbon dioxide absorbent material. During the process, in a first separation step 40, by means of the life support system 14, carbon dioxide CO₂ is separated from an ambient air of the passenger cabin 12. The carbon dioxide CO₂ is separated from the ambient air by exposing ambient air out of the passenger cabin 12 to the carbon dioxide absorbing material. For this purpose, in a separation step 42, the ion exchange resin is exposed to the ambient air by guiding part of the ambient air of the passenger cabin 12 by a fan through the ion-exchange resin that is provided in bulk form. As it flows through the bulk material, the CO₂ molecules are bound to the functional benzylamine primary groups on the outer and inner surfaces of the macro-porous resin beads, and the medium flowing through is depleted of carbon dioxide, accordingly. Subsequently, the separated fluid stream of the ambient air 14 can again be supplied to the passenger cabin 12. Subsequently, the CO₂ absorber material is regenerated after a CO₂ binding in a regeneration step 44 to remove the carbon dioxide CO₂ from the CO₂ absorber material. In the regeneration step an expulsion of the carbon dioxide CO₂ is effected. Ultimately, the expulsed carbon dioxide CO₂ is fed to a pipeline system 26.

Thereafter, conditioning of the removed carbon dioxide CO₂ takes place in the conditioning unit 30 in a conditioning step 46. In a storing step 48, the carbon dioxide CO₂ provided by the pipeline system 26 is supplied to the storage unit 20 of the cleaning system 10. The carbon dioxide CO₂ provided by means of the life support system 14 and conditioned by the conditioning unit 30 is supplied to the storage unit 20 for intermediate storage before being supplied to the work chamber 16. The storage unit 20 serves as a buffer for the cleaning process.

By means of the transfer unit 18, the carbon dioxide CO₂ is transferred into a supercritical state. In a transfer step 50 following the storing step 48, the carbon dioxide CO₂ is transferred into a supercritical state. The carbon dioxide CO₂ of life support system 14 is transferred in a supercritical state for the work chamber 16, for performing the cleaning process. For this purpose, the carbon dioxide CO₂ is compressed in a compression step 52 by means of the pump 32 to a pressure above the critical value. Subsequently, in a tempering step 54, the carbon dioxide CO₂ is heated to a temperature above the critical value by means of the heating unit 34. As is obvious to a person skilled in the art, the chronological sequence in which the compression step and heating step are performed may be implemented in a different way. For example, the heating step may be performed first. In a variation, heating step and compression step may be performed concurrently.

Furthermore, the method comprises the steps of introducing the carbon dioxide CO₂ into the work chamber 16 and performing in the work chamber 16 a purification process with the carbon dioxide CO₂ as a solvent. Therefore, subsequent to the transfer step 50, in a working step 56 the work chamber 16 is supplied with carbon dioxide CO₂ and the purification process is performed, using carbon dioxide CO₂ in a supercritical state as a solvent. The carbon dioxide CO₂ is in a supercritical state during the purification process. After the purification process, the carbon dioxide which is contaminated by the component that was extracted in the purification process (the extractant), is supplied to the carbon dioxide recovery unit 22 which, in a recovery step 58, separates pure carbon dioxide CO₂ from the contaminated carbon dioxide stream leaving the work chamber 16. The extractant thus removed may be collected and used for other suitable purposes. The separated, pure carbon dioxide CO₂ is conditioned in a second conditioning step 60 to bring it into liquid state via a cooler 38 and supplied to the storage unit 20 in a further storage step 68 via a line. The separated, pure carbon dioxide CO₂ is supplied to the storage unit 20 for reuse.

### Reference numbers

- 10: spacecraft
- 12: passenger cabin
- 14: life support system
- 16: work chamber
- 18: transfer unit
- 20: storage unit
- 22: recovery unit
- 26: pipeline system
- 28: CO₂ processing subsystem
- 30: conditioning unit
- 32: pump
- 34: heating unit
- 36: reducing valve
- 38: cooler
- 40: separation step
- 42: separation step
- 44: regeneration step
- 46: conditioning step
- 48: storing step
- 50: transfer step
- 52: compression step
- 54: heating step
- 56: working step
- 58: recovering step
- 60: second conditioning step
- 62: storing step

## Claims

1. A manned spacecraft (10),
with a life support system (14)
configured to remove metabolic carbon dioxide (CO₂) from the spacecraft's (10) internal atmosphere,
comprising a macro-porous ion-exchange resin for carbon dioxide (CO₂) absorption, and
configured to regenerate the macro-porous ion-exchange resin after a carbon dioxide (CO₂) binding, for the purpose of removing-solving the carbon dioxide (CO₂) from of the macro-porous ion-exchange resin, wherein the regeneration of the macro-porous ion exchange resin is effected by a usage of slightly overheated water vapor under atmospheric conditions and the resulting expulsion of the CO₂ or by applying a negative pressure, and wherein the carbon dioxide (CO₂), after removing from the macro-porous ion-exchange resin, is supplied to a pipeline system (26) of the life support system (14), which is intended to distribute the carbon dioxide (CO₂) in the spacecraft (10), and
with at least one work chamber (16), which is configured for a performance of an extraction and/or purification process with carbon dioxide (CO₂) as a solvent, wherein the carbon dioxide (CO₂) required for the extraction and/or purification process in the work chamber (16) is supplied by the life support system (14), and with a further work chamber (16'), which is configured for a performance of an extraction and/or purification process with carbon dioxide (CO₂) as a solvent, and which is directly connected to the life support system (14), at a distance from the work chamber (16).

2. The manned spacecraft (10) according to claim 1,
**characterized by**
at least one transfer unit (18) configured to transferring the carbon dioxide (CO₂) of the life support system (14) into a supercritical or at least near-supercritical state before being introduced into the work chamber (16).

3. The manned spacecraft (10) according to one of the preceding claims, **characterized in that**
the at least one work chamber (16) is configured for a usage of carbon dioxide (CO₂) in a supercritical or at least near-supercritical state.

4. The manned spacecraft (10) according to claim 1,
**characterized by**
at least one storage unit (20), which is arranged upstream of the work chamber (16) and is configured for an intermediate storage of carbon dioxide (CO₂).

5. The manned spacecraft (10) according to one of the preceding claims, **characterized by**
a carbon dioxide recovery unit (22), which is arranged downstream of the work chamber (16) and is configured for an at least partial separation of carbon dioxide (CO₂) from the contaminated carbon dioxide stream exiting the work chamber (16).

6. Method for operating the manned spacecraft (10) according to one of the preceding claims,
**comprising the steps of**
- separating carbon dioxide (CO₂) from ambient air by exposing the ambient air out of a passenger cabin (12) of the manned spacecraft (10) to the ion-exchange resin,
- regenerating the ion exchange resin after the CO2 binding to remove the carbon dioxide CO2 from the ion-exchange resin, for the purpose of removing-solving the carbon dioxide (CO₂) from the ion-exchange resin, wherein the regeneration of the ion-exchange resin is effected by a usage of slightly overheated water vapor under atmospheric conditions and the resulting expulsion of the CO2 or by applying a negative pressure,
- providing carbon dioxide (CO₂) by means of the pipeline system (26) of the life support system (14) to the work chamber (16) and the further work chamber (16'),
- introducing the carbon dioxide (CO₂) into one or both of the work chambers (16, 16'),
- performing in one or both of the work chambers (16, 16') an extraction and/or purification process with the carbon dioxide (CO₂) as a solvent.

7. Method according to claim 6,
**characterized in that**
the carbon dioxide (CO₂) is in a supercritical or at least near-supercritical state during the extraction and/or purification process.

## Patentansprüche

1. Bemanntes Raumschiff (10),
mit einem Lebenserhaltungssystem (14), welches dazu vorgesehen ist, metabolisches Kohlendioxid (CO₂) aus der inneren Atmosphäre des Raumschiffs (10) zu entfernen,
welches ein großporiges lonenaustauscherharz zur Absorption von Kohlendioxid (CO2) aufweist
und welches zu einer Entfernung / Lösung des Kohlendioxids (CO₂) von dem großporigen lonenaustauscherharz dazu vorgesehen ist, nach einer Bindung von Kohlendioxid (CO₂) das großporige lonenaustauscherharz zu regenerieren, wobei die Regenerierung des großporigen lonenaustauscherharzes durch Verwendung von leicht überhitztem Wasserdampf unter atmosphärischen Bedingungen mit daraus resultierendem CO₂-Ausstoß oder durch Anlegen eines Unterdrucks erfolgt
und wobei das aus dem großporigen lonenaustauscherharz entfernte Kohlendioxid (CO₂) einem Pipelinesystem (26) des Lebenserhaltungssystems (14) zugeführt wird, welches zur Verteilung des Kohlendioxids (CO₂) im Raumschiff (10) vorgesehen ist, und
mit mindestens einer Arbeitskammer (16), welche zur Durchführung eines Extraktions- und/oder Reinigungsprozesses mit Kohlendioxid (CO₂) als Lösungsmittel vorgesehen ist,
wobei das für den Extraktions- und/oder Reinigungsprozess in der Arbeitskammer (16) benötigte Kohlendioxid (CO₂) durch das Lebenserhaltungssystem (14) bereitgestellt wird, und
mit einer weiteren Arbeitskammer (16'), welche zur Durchführung eines Extraktions- und/oder Reinigungsprozesses mit Kohlendioxid (CO₂) als Lösungsmittel vorgesehen ist und welche - beabstandet von der Arbeitskammer (16) - direkt mit dem Lebenserhaltungssystem (14) verbunden ist.

2. Bemanntes Raumschiff (10) nach Anspruch 1,
**gekennzeichnet durch**
mindestens eine Transfereinheit (18), welche dazu vorgesehen ist, das Kohlendioxid (CO₂) des Lebenserhaltungssystems (14) in einen superkritischen oder zumindest annähernd superkritischen Zustand zu überführen, bevor es in die Arbeitskammer (16) eingelassen wird.

3. Bemanntes Raumschiff (10d) nach einem der vorhergehenden Ansprüche, **dadurch gekennzeichnet, dass**
die mindestens eine Arbeitskammer (16) zu einer Verwendung von Kohlendioxid (CO₂) in einem superkritischen oder zumindest annähernd superkritischen Zustand vorgesehen ist.

4. Bemanntes Raumschiff (10) nach Anspruch 1,
**gekennzeichnet durch**
mindestens eine Speichereinheit (20), welche vor der Arbeitskammer (16) angeordnet und zu einer Zwischenspeicherung von Kohlendioxid (CO₂) vorgesehen ist.

5. Bemanntes Raumschiff (10) nach einem der vorhergehenden Ansprüche, **gekennzeichnet durch**
eine Kohlendioxidrückgewinnungseinheit (22), welche nach der Arbeitskammer (16) angeordnet und zu einer zumindest teilweisen Abtrennung des Kohlendioxids (CO₂) von dem aus der Arbeitskammer (16) austretenden Strom von kontaminiertem Kohlendioxid vorgesehen ist.

6. Verfahren zum Betrieb des bemannten Raumschiffs (10) nach einem der vorhergehenden Ansprüche,
**gekennzeichnet durch die Schritte:**
- Abtrennen von Kohlendioxid (CO₂) von einer Umgebungsluft, indem die Umgebungsluft aus einer Passagierkabine (12) des bemannten Raumschiffs (10) dem lonenaustauscherharz ausgesetzt wird,
- zur Entfernung / Lösung des Kohlendioxids (CO2) von dem lonenaustauscherharz: Regenerieren des lonenaustauscherharzes nach der CO₂-Bindung, um das Kohlendioxid (CO2) von dem lonenaustauscherharz zu entfernen, wobei die Regenerierung des großporigen lonenaustauscherharzes durch Verwendung von leicht überhitztem Wasserdampf unter atmosphärischen Bedingungen mit daraus resultierendem CO₂-Ausstoß oder durch Anlegen eines Unterdrucks erfolgt,
- Zuführen von Kohlendioxid (CO₂) zu der Arbeitskammer (16) und der weiteren Arbeitskammer (16') mittels eines Pipelinesystems (26) des Lebenserhaltungssystems (14),
- Einlassen des Kohlendioxids (CO₂) in eine oder beide Arbeitskammer/n (16, 16'),
- in einer oder beiden Arbeitskammer/n (16, 16'): Durchführen eines Extraktions- und/oder Reinigungsprozesses mit dem Kohlendioxid (CO₂) als Lösungsmittel.

7. Verfahren nach Anspruch 6,
**dadurch gekennzeichnet, dass**
das Kohlendioxid (CO2) sich während des Extraktions- und/oder Reinigungsprozesses in einem superkritischen oder zumindest annähernd superkritischen Zustand befindet.

## Revendications

1. Vaisseau spatial habité (10),
avec un système de support de vie (14)
configuré à enlever du dioxyde de carbone (CO₂) métabolique de l'atmosphère intérieure du vaisseau spatial (10),
comprenant une résine à échange d'ions macroporeuse pour l'absorption de dioxyde de carbone (CO₂) et
configuré à regénérer la résine à échange d'ions macroporeuse suivant une liaison de dioxyde de carbone (CO₂) pour enlever/détacher le dioxyde de carbone (CO₂) de la résine à échange d'ions macroporeuse,
où la régénération de la résine à échange d'ions macroporeuse est effectuée en utilisant de la vapeur d'eau légèrement surchauffée sous conditions atmosphériques, ce qui résulte en expulsion du CO₂, ou en exerçant une pression négative, et
où le dioxyde de carbone (CO₂) enlevé de la résine à échange d'ions macroporeuse est fournie à un système de pipeline (26) du système de support de vie (14) qui est prévu à distribuer le dioxyde de carbone (CO₂) dans le vaisseau spatial (10), et
avec au moins une chambre à travail (16) configurée pour réaliser un procès d'extraction et/ou purification avec du dioxyde de carbone (CO₂) comme solvant, où le dioxyde de carbone (CO₂) nécessité pour le procès d'extraction et/ou purification dans la chambre à travail (16) est fourni par le système de support de vie (14), et
avec une autre chambre de travail (16') configurée pour réaliser un procès d'extraction et/ou purification avec du dioxyde de carbone (CO₂) comme solvant, qui est directement liée au système de support de vie (14) à l'écart de la chambre de travail (16).

2. Vaisseau spatial habité (10) selon la revendication 1,
**caractérisé par**
au moins une unité de transfert (18) configurée pour transférer le dioxyde de carbone (CO₂) du système de support de vie (14) dans un état supercritique ou au moins presque-supercritique avant que le dioxyde de carbone (CO₂) soit introduit dans la chambre à travail (16).

3. Vaisseau spatial habité (10) selon l'une des revendications précédentes, **caractérisé en ce que**
l'au moins une chambre à travail (16) est configurée pour l'utilisation de dioxyde de carbone (CO₂) en état supercritique ou au moins presque-supercritique.

4. Vaisseau spatial habité (10) selon la revendication 1,
**caractérisé par**
au moins une unité de stockage (20) disposée en amont de la chambre à travail (16) et configurée pour un stockage intermédiaire de dioxyde de carbone (CO₂).

5. Vaisseau spatial habité (10) selon l'une des revendications précédentes, **caractérisé par**
une unité de récupération de dioxyde de carbone (22) disposée en aval de la chambre à travail (16) et configurée pour au moins partiellement séparer le dioxyde de carbone (CO₂) du flux de dioxyde de carbone (CO₂) contaminé sortant de la chambre à travail (16).

6. Procédé en fonctionnement du vaisseau spatial habité (10) selon l'une des revendications précédentes,
**comprenant les étapes de**
- séparer du dioxyde de carbone (CO₂) de l'air ambiant en exposant de l'air ambiant d'une cabine de passager (12) du vaisseau spatial habité (10) à la résine à échange d'ions,
- regénérer la résine à échange d'ions suivant le bondage du CO₂ pour enlever le dioxyde de carbone (CO₂) de la résine à échange d'ions, dans le but d'enlever/détacher le dioxyde de carbone (CO₂) de la résine à échange d'ions,
la régénération de la résine à échange d'ions étant effectuée en utilisant de la vapeur d'eau légèrement surchauffée sous conditions atmosphériques, ce qui résulte en l'expulsion du CO₂, ou en exerçant une pression négative,
- fournir du dioxyde de carbone (CO₂) à la chambre de travail (16) et à l'autre chambre de travail (16') par le système de pipeline (26) du système de support de vie (14),
- introduire le dioxyde de carbone (CO₂) dans l'une des chambres de travail (16, 16') ou dans les deux chambres à travail (16, 16'),
- exécuter dans l'une des chambres de travail (16, 16') ou dans les deux chambres de travail (16, 16') un procès d'extraction et/ou de purification avec le dioxyde de carbone (CO₂) comme solvant.

7. Procédé selon la revendication 6,
**caractérisé en ce que**
le dioxyde de carbone (CO₂) est en état supercritique ou au moins presque-supercritique pendant le procès d'extraction et/ou de purification.
